# EUROPEAN PATENT SPECIFICATION

(11) **EP 1 355 668 B1**
(45) Date of publication and mention of the grant of the patent: **12.12.2007**
(21) Application number: 02718052.0
(22) Date of filing: 28.01.2002
(51) Int. Cl.: A61K 39/395, A61K 38/17, A61P 9/10, A61K 48/00

(54) **USE OF IL-18 INHIBITORS FOR THE TREATMENT AND/OR PREVENTION OF HEART DISEASE**
VERWENDUNG VON IL-18 HEMMERN ZUR BEHANDLUNG UND/ODER PRÄVENTION VON HERZKRANKHEITEN
UTILISATION D'INHIBITEURS D'IL-18 POUR LE TRAITEMENT ET/OU LA PREVENTION DE CARDIOPATHIES

(30) Priority: 29.01.2001 EP 01101959
(43) Date of publication of application: 29.10.2003
(73) Proprietor: Laboratoires Serono SA, 1267 Coinsins, Vaud (CH)
(72) Inventor: DINARELLO, Charles, Boulder, CO 80302 (US); POMERANTZ, Benjamin, University City, MO 63130 (US); REZNIKOV, Leonid, Denver, CO 80224 (US); HARKEN, Alden, Littleton, CO 80121 (US); CHVATCHKO, Yolande, CH-1232 Confignon (CH)
(74) Representative: Merck Serono International S.A. Intellectual Property
(86) International application number: PCT/EP2002/000844
(87) International publication number: WO 2002/060479

(56) References cited:
- WO-A-01/03719
- WO-A-01/58956
- WO-A-98/24804
- WO-A-99/09063
- D. AFFLECK ET AL.: "Interleukin-18 production following murine cardiac transplantation: correlation with histiologic rejection and the induction of IFN-gamma." JOURNAL OF INTERFERON AND CYTOKINE RESEARCH, vol. 21, no. 1, January 2001 (2001-01), pages 1-9, XP001002506 New York, NY, USA
- Y. SETA ET AL.: "Interleukin-18 in acute myocardial infarction." HEART, vol. 84, no. 6, December 2000 (2000-12), page 668 XP001002516 London, GB
- T. KANDA ET AL.: "Effect of interleukin-18 on viral myocarditis: enhancement of interferon-gamma and natural killer cell activity." JOURNAL OF MOLECULAR AND CELLULAR CARDIOLOGY, vol. 32, no. 12, December 2000 (2000-12), pages 2163-2171, XP001002525 London, GB
- C. DINARELLO: "Interleukin-18." METHODS, vol. 19, no. 1, September 1999 (1999-09), pages 121-132, XP002168537 USA
- B. POMERANTZ ET AL.: "Inhibition of caspase 1 reduces human myocardial ischemic dysfunction via inhibition of IL-18 and IL-1beta." PROCEEDINGS OF THE NATIONAL ACADEMY OF SCIENCES OF THE U.S.A., vol. 98, no. 5, 27 February 2001 (2001-02-27), pages 2871-2876, XP002168538 Washington, DC, USA
- USHINO ET AL: "Cloning o fthe cDNA for human IFN-gama-inducing factor, expression in Escherichia coli, and studies on the biologic activities of the protein" J. IMMUNOLOGY, vol. 156, 1996, pages 4274-4279,
- FAUCI: "Harrison's principles of internal medicine" 1994, INTERNATIONAL EDITION MC GRAW HILL
- MACSWEEN AND WHALEY: "Muir's texbook of pathology" 1992, EDWARD ARNOLD
- HEGEWISCH: "TNF-Induced cardiomyopathy" THE LANCET, vol. 335, no. 8684, 1990, pages 294-295,
- YOKOYAMA ET AL: "Tumor necrosis factor-alpha provokes a hypertrophic growth response in adult cardiac myocytes" CIRCULATION, vol. 95, 1997, pages 1247-1252,

## Description

### FIELD OF THE INVENTION

The present invention is in the field of cardiovascular diseases. More specifically, it relates to the use of an inhibitor of IL-18 for the treatment and/or prevention of cardiomyopathy.

### BACKGROUND OF THE INVENTION

The cytokine interleukin 18 (IL-18) was initially described as an interferon-γ (IFN-y) inducing factor (Nakamura et al., 1989). It is an early signal in the development of T-lymphocyte helper cell type 1 (TH1) responses. IL-18 acts together with IL-12. IL-2, antigens, mitogens, and possibly further factors, to induce the production of IFN-γ. IL-18 also enhances the production of GM-CSF and IL-2, potentiates anti-CD3 induced T cell proliferation, and increases Fas-mediated killing of natural killer cells.

Mature IL-18 is produced from its precursor by the IL-1β converting enzyme (ICE, caspase-1).

The IL-18 receptor consists of at least two components, co-operating in ligand binding. High- and low-affinity binding sites for IL-18 were found in Murine IL-12 stimulated T cells (Yoshimoto et al., 1998), suggesting a multiple chain receptor complex. Two receptor subunits have been identified so far, both belonging to the IL-1 receptor family (Pamet et al., 1996; Kim et al., 2001). The signal, transduction of IL-18 Involves activation of NF-κB (DiDonato et al., 1997). The IL-18 receptor complex consists of two receptor chains: a ligand-binding chain termed the IL-18Rα chain and a signal-transducing chain termed the IL-18Rβ chain. The IL-18R chain was initially isolated as a cell surface protein binding to radiolabeled IL-18; the protein was purified and its amino acid sequence revealed identity with a previously reported orphan receptor termed the IL-1R-related protein (IL-1Rrp) (Torigoe et al., 1997).

Recently, a soluble protein having a high affinity for IL-18 has been isolated from human urine, and the human and mouse cDNAs as well as the human gene were cloned (Novick et al., 1999; WO 99/09063). The protein has been designated IL-18 binding protein (IL-18BP).

IL-18BP is not the extracellular domain of one of the known IL18 receptors, but a secreted, naturally circulating protein. It belongs to a novel family of secreted proteins, further including several Poxvirus-encoded proteins (Novick et al., 1999). Urinary as well as recombinant IL-18BP specifically bind IL-18 with a high affinity and modulate the biological affinity of IL-18.

The IL-18BP gene was localised to the human chromosome 11q13, and no exon coding for a transmembrane domain was found in an 8.3kb genomic sequence. Four splice variants or isoforms of IL-18BP generated by alternative mRNA splicing have been found in humans so far. They were designated IL-18BP a, b, c and d, all sharing the same N-terminus and differing in the C-terminus (Novick et al, 1999). These isoforms vary in their ability to bind IL-18. Of the four, hIL-18BP isoforms a and c are known to have a neutralising capacity for IL-18. Human IL-18BP isoform a cross-reacts with murine IL-18.

Heart diseases are defined as disorders that affect the heart muscle or the blood vessels of the heart (The Merck Manual Home Edition, www.merck.com). A vascular disorder is a blood vessel problem such as poor circulation caused by blockage. Heart diseases are also called cardiovascular disorders.

Ischemic heart disease is a common cause of cardiac failure and it is the most frequent cause of death in Western societies. It is usually due to coronary artery atheroma. Myocardial lesions include ischemic fibrosis and acute infarction. Under normal conditions, the blood flow in coronary arteries is closely matched to the metabolic demands of cardiac muscle. Ischemic heart disease results when the blood supply becomes insufficient, because either the blood supply itself is impaired or the myocardium becomes hypertrophic and makes a greater demand on the blood supply. Coronary blood flow is normally independent on aortic pressure. An efficient autoregulatory mechanism exists to control the blood flow through the coronary vascular bed.

When an obstruction develops in a major coronary artery, usually because of atherosclerosis or ateriosclerosis, coronary blood flow is initially preserved, because peripheral resistance distal to the obstruction is reduced. When the vessel lumen is more than 75% occluded, ischemia develops, particularly if the coronary collateral circulation is poorly developed.

Cardiac muscle is extremely active metabolically, and mitochondria constitute over 30% of the volume of individual fibres. Aerobic metabolism is essential, as there are very poor reserves of high-energy phosphates. Cardiac muscle death occurs when tissue adenosine triphosphate (ATP) levels are very low and when anaerobic glycolysis has virtually ceased. As with other tissues, the precise cause of death is uncertain, but lethal cardiac muscle injuries are associated with membrane damage and the sudden entry of calcium into the cell cytoplasm. After brief periods of ischemia cardiac blood flow can be re-established (reperfusion). However, after a critical interval reperfusion is impossible, probably as a result of swelling of capillary endothelial cells.

Atherosclerosis accounts for the vast majority of coronary artery disease. Ischemic heart disease can also result from low coronary arterial perfusion. Stoke, especially as a result of hemorrhage, is a frequent cause of this.

As pointed out above, ischemic heart disease is caused by an imbalance between the myocardial blood flow and the metabolic demand of the myocardium. Blood flow can be further decreased by superimposed events such as vasospasm, thrombosis, or circulatory changes leading to hypoperfusion.

Coronary artery perfusion depends upon the pressure differential between the ostia (aortic diastolic pressure) and coronary sinus (right atrial pressure). Coronary blood flow is reduced during systole because of Venturi effects at the coronary orifices and compression of intramuscular arteries during ventricular contraction. Factors reducing coronary blood flow include decreased aortic diastolic pressure, Increased intraventricular pressure and myocardial contraction, coronary artery stenosis, aortic valve stenosis and regurgitation and Increased right atrial pressure.

Thrombolytic therapy with agents such as streptokinase or tissue plasminogen activator (TPA) is often used to lyse a recently formed thrombus. Such therapy with lysis of the thrombus can re-establish blood flow in a majority of cases. This helps to prevent significant myocardial injury, if early (less than an hour or so) in the course of events, and can at least help to reduce further damage.

Angina pectoris is a symptom complex of ischemic heart disease characterized by paroxysmal attacks of chest pain, is usually substernal or precordial. It is caused by myocardial ischemia that falls short of inducing infarction. Sudden cardiac death may occur, which is the unexpected death from cardiac causes usually within one hour after a cardiac event or without the onset of symptoms. It strikes 300,000 - 400,000 persons annually.

Other forms of heart disease include alcoholic cardiomyopathy, aortic valve prolapse, aortic valve stenosis, arrhythmias, cardiogenic shock, congenital heart disease, dilated cardiomyopathy, heart attack, heart failure, heart tumor, heart valve pulmonary stenosis, hypertrophic cardiomyopathy, idiopathic cardiomyopathy, ischemic heart disease, ischemic cardiomyopathy, mitral regurgitation, mitral valve prolapse, peripartum cardiomyopathy, stable angina.

Myocardial infarction is a further form of ischemic heart disease. The pathogenesis can include occlusive intracoronary thrombus, i.e. a thrombus overlying an ulcerated or fissured stenotic plaque. Occlusive intracoronary thrombus causes 90% of transmural acute myocardial infarctions. Vasospasm may be with or without coronary atherosclerosis and possible association with platelet aggregation. Emboli may also be present in myocardial infarction.

The gross morphologic appearance of a myocardial infarction can vary. Transmural infarct involves the entire thickness of the left ventricular wall from endocardium to epicardium. Subendocardial infarct involves multifocal areas of necrosis confined to the inner 1/3-1/2 of the left ventricular wall. Complications of myocaridal infarctions can include arrhythmias and conduction defects, with possible "sudden death", extension of infarction, or re-infarction, congestive heart failure (pulmonary edema), cardiogenic shock, pericarditis, mural thrombosis, with possible embolization, myocardial wall rupture, with possible tamponade, papillary muscle rupture, with possible valvular insufficiency, ventricular aneurysm formation.

Myocardial infarction (MI) is defined as an ischemic myocardial necrosis usually resulting from abrupt reduction in coronary blood flow to a segment of myocardium.

In > 90% of patients with acute MI, an acute thrombus, often associated with plaque rupture, occludes the artery (previously partially obstructed by an atherosclerotic plaque) that supplies the damaged area. Altered platelet function induced by endothelial change in the atherosclerotic plaque presumably contributes to thrombogenesis. Spontaneous thrombolysis occurs in about 2/3 of patients so that, 24 h later, thrombotic occlusion is found in only about 30%.

Myocardial infarction is sometimes caused by arterial embolization (e.g. in mitral or aortic stenosis, infective endocarditis, and marantic endocarditis). Myocardial infarction has been reported in patients with coronary spasm and otherwise normal coronary arteries. Cocaine causes intense coronary arterial spasm, and users may present with cocaine-induced angina or myocardial infarction. Autopsy studies and coronary angiography have shown that cocaine-induced coronary thrombosis may occur in normal coronary arteries or be superimposed on preexisting atheroma.

Myocardial infarction is predominantly a disease of the left ventricle, but damage may extend into the right ventricle (RV) or the atria. Right ventricle infarction usually results from occlusion of the right coronary or a dominant left circumflex artery and is characterized by high right ventricle filling pressure, often with severe tricuspid regurgitation and reduced cardiac output. Some degree of right ventricle dysfunction occurs in about half of patients with an inferior-posterior infarction, producing hemodynamic abnormality in 10 to 15%.

The ability of the heart to continue functioning as a pump relates directly to the extent of myocardial damage.

Transmural infarcts involve the whole thickness of myocardium from epicardium to endocardium and are usually characterized by abnormal Q waves on ECG. Nontransmural or subendocardial infarcts do not extend through the ventricular wall and cause only ST segment and T-wave abnormalities. Subendocardial infarcts usually involve the inner 1/3 of the myocardium where wall tension is highest and myocardial blood flow is most vulnerable to circulatory changes. They may also follow prolonged hypotension. Because the transmural depth of necrosis cannot be precisely determined clinically, infarcts are better classified by ECG as Q wave and non-Q wave. The volume of myocardium destroyed can be estimated by the extent and duration of CK elevation.

Ischemic cardiomyopathy is another disease within ischemic heart disease. In this condition, there may be previous myocardial infarction, but the disease results from severe coronary atherosclerosis involving all major branches. The result is an inadequate vascular supply, which leads to myocyte loss. The myocyte loss coupled with fibrosis in the form of interstitial collagen deposition results in decreased compliance, which along with the accompanying cardiac dilation, results in overload of remaining myocytes. This keeps the process going, with compensation by continuing myocyte hypertrophy. There may even be compensation through hyperplasia as well as hypertrophy, which can explain the enormous size (2 to 3 times normal size) of the resulting heart. Eventually, the heart can no longer compensate, and cardiac failure ensues with arrhythmias and/or ischemic events. Thus, clinically, there is slow, progressive heart failure with or without a history of a previous myocardial infarction or anginal pain. ischemic cardiomyopathy is responsible for as much as 40% of the mortality in ischemic heart disease.

During ischemia as well as reperfusion of the heart, numerous endogenous mediators, such as small molecule second messengers, are produced which affect myocardial function. Within minutes of an ischemic episode, myocardial contractile force diminishes and the overall recovery of contractile force is largely dependent on the duration of the ischemic period (Daemen et al., 1999). For example, during an ischemic event, Ca²⁺ homeostasis is perturbed, oxygen-derived free radicals are generated and nitric oxide (NO) synthesis and release takes place. In addition, there is also local production of cytokines, particularly TNFα and IL-1β (Bolli, 1990). In the intact heart, these cytokines contribute to ischemia-induced myocardial dysfunction by inducing gene expression for inducible NO synthase (iNOS)(Daemen et al., 1999), cyclooxygenase-2 (COX-2) and phospholipase A2 as well as vascular adhesion molecules and several chemokines. As a result, there is immediate depression of myocardial contractile force mediated by small molecule messengers followed by cytokine-mediated neutrophil infiltration, that further damages heart muscle Animal hearts studied in the absence of blood or blood products elaborate TNFα (Herskowitz et al., 1995) and IL-1β during an ischemic challenge. Cardiomyocytes also lose contractile force due to the action of these endogenous cytokines (Meldrum et al., 1998).

Most of the experimental data concerning TNFα and IL-1β mediated myocardial dysfunction are derived from animal studies. However, human myocardial tissues obtained from patients undergoing elective cardio-pulmonary bypass procedures have been studied under controlled, ex vivo conditions (Gurevitch et al., 1996; Cleveland et al., 1997). In this experimental model, human atrial trabeculae are suspended in a blood-free, physiologically oxygenated buffer bath and then exposed to an episode of simulated ischemia. During this time, contractile force decreases dramatically; when the tissue is re-exposed to oxygen, the contractile force returns but is diminished (60-70% reduction) and evidence of myocardial damage is observed by release of creatine kinase (CK) (Gurevitch et al., 1996; Cleveland et al., 1997). When TNF bioactivity is specifically neutralized during ischemia/reperfusion (I/R), a greater return of contractile force is observed suggesting that endogenous myocardial TNF activity contributes to the contractile dysfunction induced by the ischemic event (Cain et al., 1999).

Daemen et al. (1999) have studied tissue injury as a consequence of ischemia followed by reperfusion employing a murine model of renal ischemia. They showed that renal IL-18 mRNA up-regulation coincided with caspase-1 activation at day 1 following ischemia. IFN-γ and IL-12 mRNA were subsequently up-regulated at day 6 following ischemia. Combined, but not separate, *in vivo* neutralization of the IFN-γ inducing cytokines IL-12 and IL-18 reduced IFN-gamma-dependent MHC class I and II up-regulation to a similar extent as IFN-γ neutralization. IL-18 inhibitors and their involvement in diseases have been described in the prior art. For instance, WO 98/24804 discloses interleukin-1β converting enzyme (ICE) inhibitors. This document further indicates that ICE may play a role in programmed cell death or apoptosis, and that a therapeutic application of inhibition of apoptosis may include treatment of myocardial infarction. WO 01/58956 describes anti-IL-18 antibodies. Among many different hypothetical therapeutic uses of these antibodies, heart failure, myocardial infarction and cardiomyopathy are mentioned. WO 01/85201 relates to the use of IL-18 inhibitors in atherosclerosis. Seta et al., Heart 2000; 84: 668-669 describes the involvement of IL-18 in acute myocardial infarction and suggests IL-18 as a marker of cardiac damage in patients with acute myocardial infarction. WO 01/03719 describes osteoprotegerin, a member of the tumor necrosis factor receptor superfamily, and its involvement in the regulation of bone metabolism. This document also refers to combination therapy with a number of compounds, among which IL-18 inhibitors and TNF-inhibitors are mentioned. Heart diseases are not mentioned in this document.

However, IL-18 has not been described to play a role in cardiomyopathy so far.

### SUMMARY OF THE INVENTION

The invention is based on the finding that an inhibitor of IL-18 substantial improved the contractile function of heart in an ischemia/reperfusion model of suprafused human atrial myocardium. Inhibition of caspase-1 (ICE) also attenuated the depression in contractile force following ischemia and reperfusion.

Furthermore, the administration of an IL-18 inhibitor in a murine model of myocardial infarction resulted in an enhanced survival and in significant improvement of ventricular function.

These studies demonstrate that inhibitors of IL-18 are suitable for treatment or prevention of myocardial dysfunction.

The present invention therefore relates to the use of an Inhibitor of IL-18 in the manufacture of a medicament for treatment and/or prevention of cardiomyopathy.

In order to apply a gene therapeutic approach to deliver the IL-18 inhibitor to the diseased tissue or cell, the Invention further relates to the use an expression vector comprising the coding sequence of an IL-18 inhibitor for the treatment and/or prevention of cardiomyopathy.

### BRIEF DESCRIPTION OF THE DRAWINGS

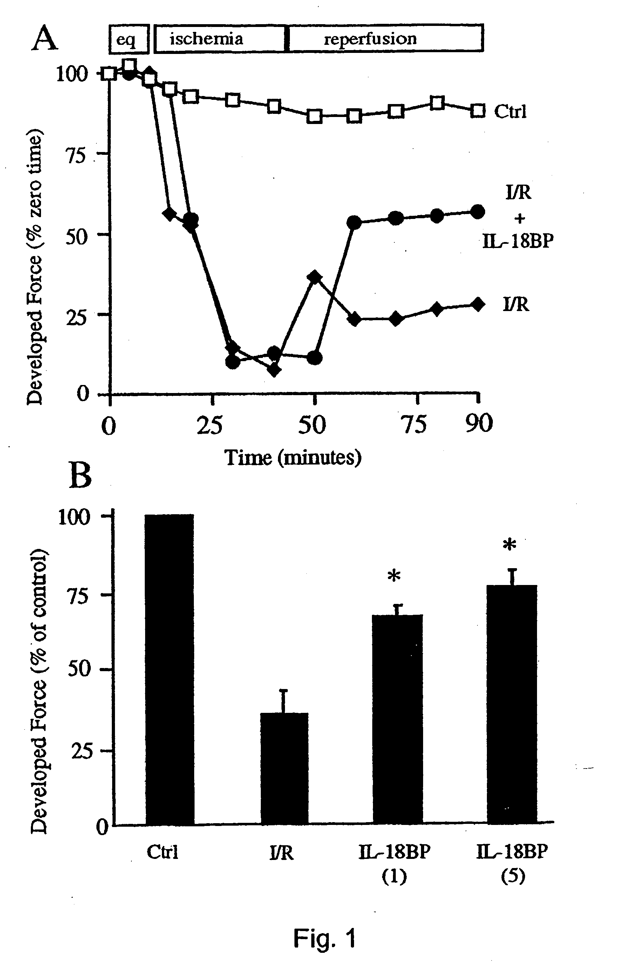

- Fig. 1: shows the effect of IL-18BP on ischemia-induced myocardial contractile dysfunction.
(A) Kinetic response to ischemic injury. Following equilibration (eq), control (Ctrl) trabeculae were suprafused under normoxic conditions throughout the experiment. Trabeculae were subjected to ischemia/reperfusion in the absence or presence of IL-18BP (5 µg/ml). The vertical axis indicates percent of developed force compared to initiation of the experiment (time zero). The data are derived from trabeculae of a single patient and are representative of the methods used to calculate the mean change in developed force at 90 minutes.
(B) Post-ischemic developed force following neutralization of IL-18 with 1 or 5 µg/ml of IL-18BP. Results are expressed as mean percent changes in developed force relative to Ctrl following completion of reperfusion (90 minutes). Numbers in parentheses indicate IL-18BP in µg/ml. N=6. * p<0.01 compared to I/R (ischemia/reperfusion).

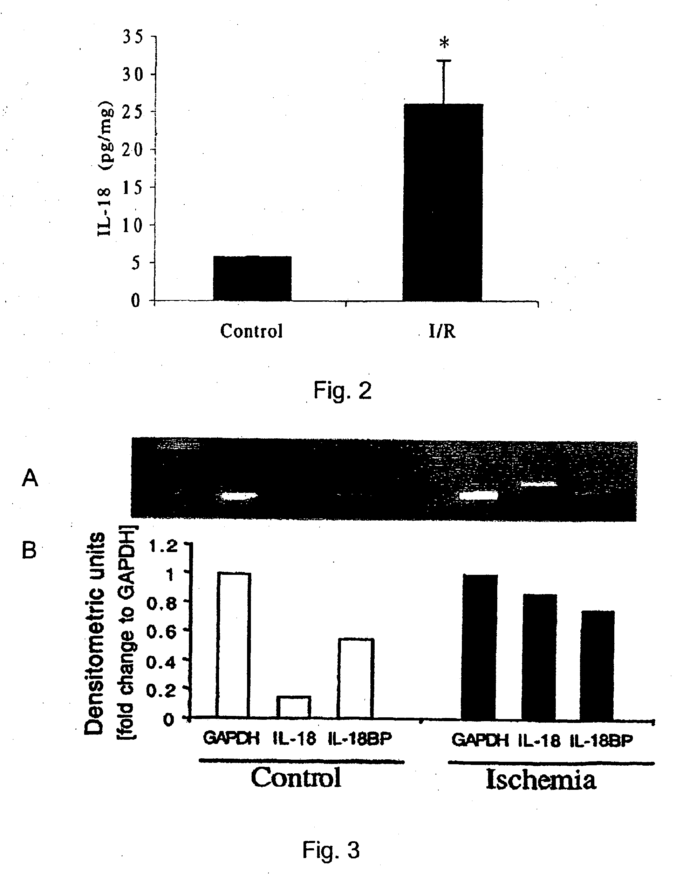

- Fig. 2:: shows the myocardial IL-18 protein content. Trabeculae were homogenized following 90 minutes of suprafusion under normoxic conditions (control) or 45 minutes following 30 minutes of ischemia (I/R). Trabeculae were matched from the same subjects. IL-18 levels are indicated on the vertical axis in pg/ml. N=4.* p<0.01.
- Fig. 3:: shows the steady state IL-18 and IL-18BP mRNA levels in control and ischemic atrial tissue. Levels of IL-18 and IL-18BP mRNA were determined by RT-PCR. Data are from one of two subjects evaluated. A shows the ethidiumbromide stained agarose gel, in which the PCR products were separated, and B shows the results of quantification of the amount of PCT product as fold change to control (GAPDH).

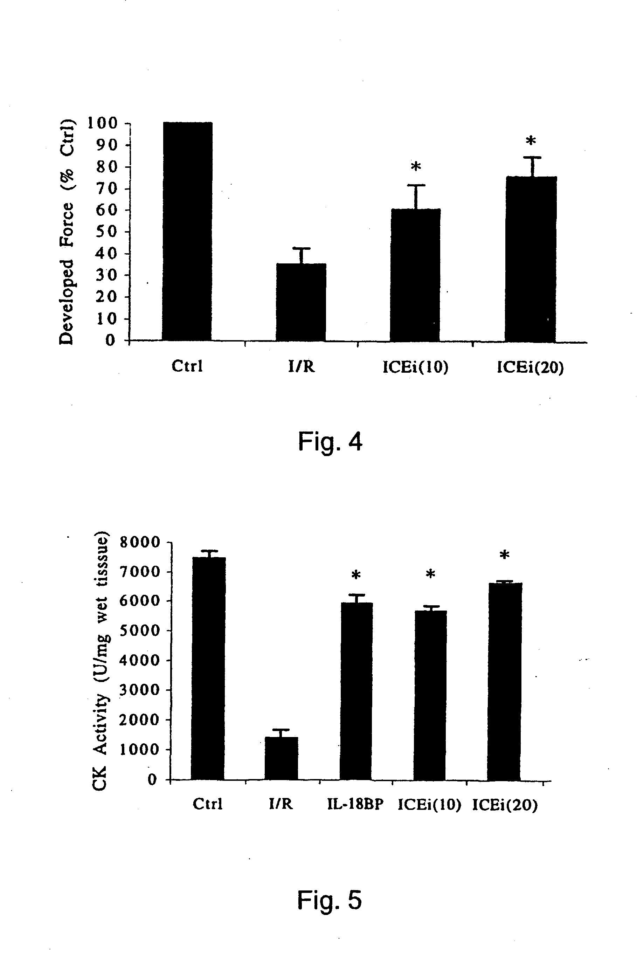

- Fig 4:: shows the effect of ICE inhibition on post-ischemic developed force. Results are expressed as mean percent change in developed force relative to control (Crtl) following ischemia/reperfusion (I/R). Numbers in parentheses indicate the concentration of ICEi in µg/ml. N=7. * p<0.01 compared to I/R.
- Fig. 5:: shows the tissue creatine kinase (CK) activity following I/R. CK is expressed in units of activity per milligram wet weight of tissue. The experimental conditions are indicated under the horizontal axis. Ctrl and I/R, N=6; IL-18BP (5 µg/ml), N=5; ICEi (10 and 20 µg/ml), N=5 each; * p<0.05 compared to I/R.

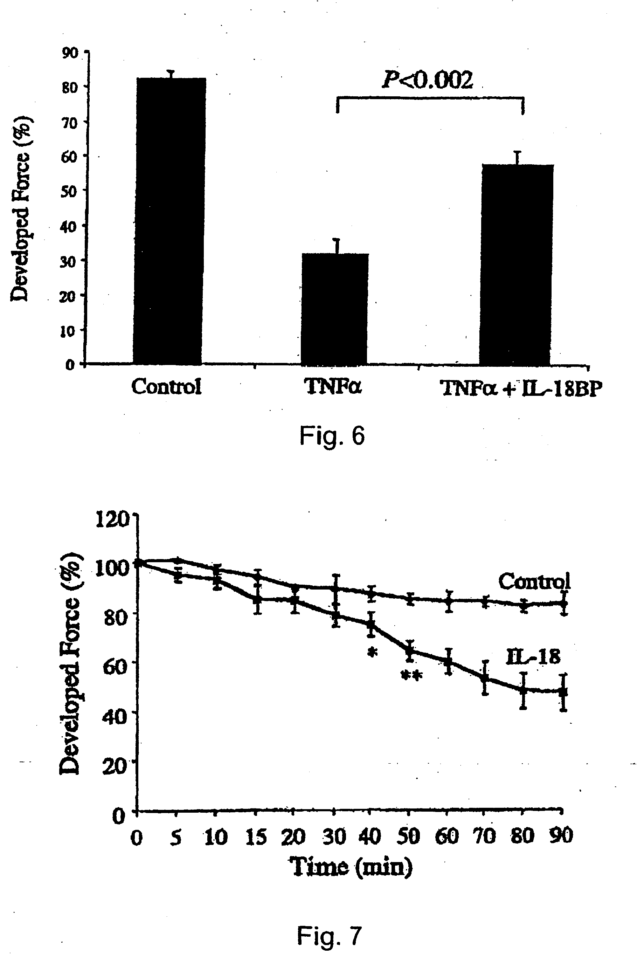

- Fig. 6:: shows the mean change in developed force relative to the developed force following the equilibration period, set at 100% (n=5), of trabeculae incubated with 10 µg/ml for 15 min, prior to the addition of TNFα (1 ng/ml). TNFα and IL-18BP were added to each bath change.
- Fig. 7:: shows the temporal response of human atrial trabeculae to IL-18 under normoxic conditions. Mature IL-18 (100 ng/ml) were added to atrial trabeculae throughout the 90 min experimental period. The vertical axis indicates the mean percent change from baseline developed force. The baseline was determined at the end of the equilibration period (not shown). (n=6). * P < 0.05, ** P < 0.001 as compared to control at the same interval and for the remainder of the experimental period.

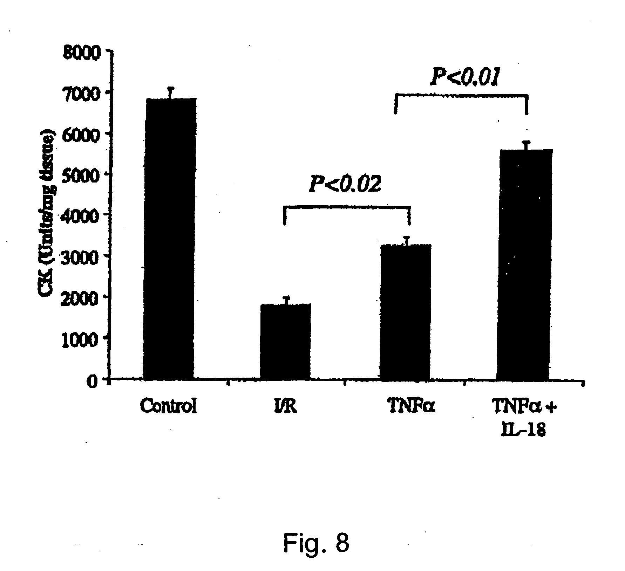

- Fig. 8:: shows the preservation of myocellular tissue creatine kinase activity following exposure to I/R. TNFα (1 ng/ml) and TNFα (10 ng/ml) + IL-18BP. CK activity is expressed in units of CK activity per milligram of wet tissue weight. (n=6).

### DETAILED DESCRIPTION OF THE INVENTION

The present invention is based on the finding that IL-18 inhibitors exert a beneficial effect In heart diseases, in particular in ischemic heart diseases. As shown in the examples below, several different IL-18 inhibitors were shown to exhibit a significant beneficial effect on post-ischemic developed force of the heart muscle.

In addition to this, an inhibitor of IL-18 was tested in an *in vivo* model of myocardial infarction and resulted in an elevated survival and significantly improved ventricular function.

The invention therefore relates to the use of an IL-18 inhibitor for the manufacture of a medicament for the treatment and/or prevention of cardiomyopathy.

Cardiomyopathy is any structural or functional abnormality of the ventricular myocardium.

The term "prevention" within the context of this invention refers not only to a complete prevention of a certain effect, but also to any partial or substantial prevention, attenuation, reduction, decrease or diminishing of the effect before or at early onset of disease.

The term "treatment" within the context of this invention refers to any beneficial effect on progression of disease, including attenuation, reduction, decrease or diminishing of the pathological development after onset of disease.

The term "inhibitor of IL-18* within the context of this invention refers to any molecule modulating IL-18 production and/or action In such a way that IL-18 production and/or action is attenuated, reduced, or partially, substantially or completely prevented or blocked.

Inhibitors of IL-18 action may be IL-18 antibodies, such as polyclonal or monoclonal antibodies

In the present invention, the inhibitor of IL-18 is selected from inhibitors of caspase-1 (ICE), antibodies directed against IL-18, antibodies directed against any of the IL-18 receptor subunits, and IL-18 binding proteins, isoforms, muteins, fused proteins, functional derivatives thereof inhibiting the biological activity of IL-18.

The term "IL-18 binding proteins" is used herein synonymously with "IL-18 binding protein" or "IL18BP". It comprises IL-18 binding proteins as defined in WO 99/09063 or in Novick et al., 1999, including splice variants and/or isoforms of IL-18 binding proteins, as defined in Kim et al., 2000, which bind to IL-18. in particular, human isoforms a and c of IL-18BP are useful in accordance with the presence invention. The proteins useful according to the present invention may be glycosylated or non-glycosylated, they may be derived from natural sources, such as urine, or they may preferably be produced recombinantly. Recombinant expression may be carried out in prokaryotic expression systems like E. coli, or in eukaryotic, and preferably in mammalian, expression systems.

As used herein the term "muteins" refers to analogs of an IL-18BP, or analogs of a viral IL-18BP, in which one or more of the amino acid residues of a natural IL-18BP or viral IL-18BP are replaced by different amino acid residues, or are deleted, or one or more amino add residues are added to the natural sequence of an IL-18BP, or a viral IL-18BP. without changing considerably the activity of the resulting products as compared with the wild type IL-18BP or viral IL-18BP. These muteins are prepared by known synthesis and/or by site-directed mutagenesis techniques, or any other known technique suitable therefor.

Muteins in accordance with the present invention include proteins encoded by a nucleic acid, such as DNA or RNA, which hybridizes to DNA or RNA, which encodes an IL-18BP or encodes a viral IL-18BP, in accordance with the present invention, under stringent conditions. The term "stringent conditions" refers to hybridization and subsequent washing conditions, which those of ordinary skill in the art conventionally refer to as "stringent". See Ausubel et al., Current Protocols in Molecular Biology, supra, Interscience, N.Y., §§6.3 and 6.4 (1987, 1992), and Sambrook et al., supra. Without limitation, examples of stringent conditions include washing conditions 12-20°C below the calculated Tm of the hybrid under study in, e.g., 2 x SSC and 0.5% SDS for 5 minutes, 2 x SSC and 0.1% SDS for 15 minutes; 0.1 x SSC and 0.5% SDS at 37°C for 30-60 minutes and then, a 0.1 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, it is preferable to use tetramethyl ammonium chloride (TMAC) instead of SSC. See Ausubel, supra.

Any such mutein preferably has a sequence of amino acids sufficiently duplicative of that of an IL-18BP, or sufficiently duplicative of a viral IL-18BP, such as to have an activity comparable to IL-18BP. One activity of IL-18BP is its capability of binding IL-18. As long as the mutein has substantial binding activity to IL-18, it can be used in the purification of IL-18, such as by means of affinity chromatography, and thus can be considered to have substantially similar activity to IL-18BP. Thus, it can be determined whether any given mutein has substantially the same activity as IL-18BP by means of routine experimentation comprising subjecting such a mutein, e.g., to a simple sandwich competition assay to determine whether or not it binds to an appropriately labeled IL-18, such as radioimmunoassay or ELISA assay.

In a preferred embodiment, any such mutein has at least 40% identity or homology with the sequence of either an IL-18BP or a virally-encoded IL-18BP homologue, as defined in WO 99/09063. More preferably, it has at least 50%, at least 60%, at least 70%, at least 80% or, most preferably, at least 90% identity or homology thereto.

Muteins of IL-18BP polypeptides or muteins of viral IL-18BPs, which can be used in accordance with the present invention, or nucleic add coding therefor, include a finite set of substantially corresponding sequences as substitution peptides or polynucleotides which can be routinely obtained by one of ordinary skill in the art, without undue experimentation, based on the teachings and guidance presented herein.

Preferred changes for muteins in accordance with the present invention are what are known as "conservative" substitutions. Conservative amino acid substitutions of IL-18BP polypeptides or proteins or viral IL-18BPs, may include synonymous amino adds within a group which have sufficiently similar physicochemical properties that substitution between members of the group will preserve the biological function of the molecule (Grantham, 1974). It is clear that insertions and deletions of amino acids may also be made in the above-defined sequences without altering their function, particular1y if the insertions or deletions only involve a few amino acids, e.g., under thirty, and preferably under ten, and do not remove or displace amino acids which are critical to a functional conformation, e.g., cysteine residues. Proteins and muteins produced by such deletions and/or insertions come within the purview of the present invention.

Preferably, the synonymous amino acid groups are those defined in Table 1. More preferably, the synonymous amino add groups are those defined in Table 2; and most preferably the synonymous amino acid groups are those defined in Table 3.

**TABLE 1 Preferred Groups of Synonymous Amino Acids**

| Amino Acid | Synonymous Group |
|---|---|
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gin | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**TABLE 2 More Preferred Groups of Synonymous Amino Acids**

| Amino Acid | Synonymous Group |
|---|---|
| Ser | Ser |
| Arg | His, Lys, Arg |
| Leu | Leu, lle, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE 3 Most Preferred Groups of Synonymous Amino Acids**

| Amino Acid | Synonymous Group |
|---|---|
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, lle, Leu |
| Trp | Met |

Examples of production of amino acid substitutions in proteins which can be used for obtaining muteins of IL-18BP polypeptides or proteins, or muteins of viral IL-18BPs, for use in the present invention include any known method steps, such as presented in US patents 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al).

The term "fused protein" refers to a polypeptide comprising an IL-18BP, or a viral IL-18BP, or a mutein or fragment thereof, fused with another protein, which, e.g., has an extended residence time in body fluids. An IL-18BP or a viral IL-18BP, may thus be fused to another protein, polypeptide or the like, e.g., an immunoglobulin or a fragment thereof.

"Functional derivatives" as used herein cover derivatives of IL-18BPs or a viral IL-18BP, and their muteins and fused proteins, which may be prepared from the functional groups which occur as side chains on the residues or the N- or C-terminal groups, by means known in the art, and are included in the invention as long as they remain pharmaceutically acceptable, i.e. they do not destroy the activity of the protein which is substantially similar to the activity of IL-18BP, or viral IL-18BPs, and do not confer toxic properties on compositions containing it.

These derivatives may, for example, include polyethylene glycol side-chains, which may mask antigenic sites and extend the residence of an IL-18BP or a viral IL-18BP in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

In a further preferred embodiment of the invention, the inhibitor of IL-18 is antibody directed against IL-18 or its receptor, the IL-18R. Antibodies directed to any of the IL-18R subunits, called IL-18Rα and β, may be used in accordance with the present invention.

The antibodies according to the invention may be polyclonal or monoclonal, chimeric, humanized, or even fully human. Recombinant antibodies and fragments thereof are characterized by high affinity binding to IL-18 or IL-18R *in vivo* and low toxicity. The antibodies which can be used in the invention are characterized by their ability to treat patients for a period sufficient to have good to excellent regression or alleviation of the pathogenic condition or any symptom or group of symptoms related to a pathogenic condition, and a low toxicity.

Neutralizing antibodies are readily raised in animals such as rabbits, goat or mice by immunization with IL-18 or IL-18Rα or β. Immunized mice are particularly useful for providing sources of B cells for the manufacture of hybridomas, which In turn are cultured to produce large quantities of anti-IL-18 monoclonal antibodies.

Chimeric antibodies are immunoglobulin molecules characterized by two or more segments or portions derived from different animal species. Generally, the variable region of the chimeric antibody is derived from a non-human mammalian antibody, such as murine monoclonal antibody, and the immunoglobulin constant region is derived from a human immunoglobulin molecule. Preferably, both regions and the combination have low immunogenicity as routinely determined (Elliott et al., 1994). Humanized antibodies are immunoglobulin molecules created by genetic engineering techniques in which the murine constant regions are replaced with human counterparts while retaining the murine antigen binding regions. The resulting mouse-human chimeric antibody preferably have reduced immunogenicity and improved pharmacokinetics in humans (Knight et al., 1993).

Thus, in a further preferred embodiment, IL-18 or IL-18R antibody is a humanized antibody. Preferred examples of humanized anfi-IL-18 antibodies are described in the European Patent Application EP 0 974 600, for example.

In yet a further preferred embodiment, the antibody is fully human. The technology for producing human antibodies is described in detail e.g. in WO00/76310, WO99/53049, US 6.162,963 or AU5336100.

One method for the preparation of fully human antibodies consist of "humanization" of the mouse humoral immune system, i.e. production of mouse strains able to produce human lg (Xenomice), by the introduction of human immunoglobulin (Ig) lod into mice in which the endogenous lg genes have been inactivated. The lg loci are complex in terms of both their physical structure and the gene rearrangement and expression processes required to ultimately produce a broad immune response. Antibody diversity is primarily generated by combinatorial rearrangement between different V, D, and J genes present in the lg loci. These loci also contain the interspersed regulatory elements, which control antibody expression, allelic exclusion, class switching and affinity maturation. Introduction of un-rearranged human lg transgenes into mice has demonstrated that the mouse recombination machinery is compatible with human genes. Furthermore, hybridomas secreting antigen specific hu-mAbs of various isotypes can be obtained by Xenomice immunisation with antigen.

Fully human antibodies and methods for their production are known In the art (Mendez et al (1997); Buggemann et al (1991); Tomizuka et al.. (2000) Patent WO 98/24893).

In a highly preferred embodiment of the present invention, the inhibitor of IL-18 is an IL-18BP, or an isoform, a mutein, fused protein, functional derivative thereof. These isoforms, muteins, fused proteins or functional derivatives retain the biological activity of IL-18BP, in particular the binding to IL-18, and preferably have essentially at least an activity similar to IL-18BP. Ideally, such proteins have an enhanced biological activity as compared to unmodified IL-18BP.

The sequences of IL-18BP and its splice variants/isoforms can be taken from WO99/09063 or from Novick et al., 1999, as well as from Kim et al., 2000.

Functional derivatives of IL-18BP may be conjugated to polymers in order to improve the properties of the protein, such as the stability, half-life, bioavailability, tolerance by the human body, or immunogenicity. To achieve this goal, IL18-BP may be linked e.g. to Polyethlyenglycol (PEG). PEGylation may be carried out by known methods, described in WO 92/13095, for example.

Therefore, in a preferred embodiment of the present invention, the inhibitors of IL-18, and in particular the IL-18BP is PEGylated.

In a further preferred embodiment of the invention, the inhibitor of IL-18 comprises an immunoglobulin fusion, i.e. the inhibitor of IL-18 is a fused protein comprising all or part of an IL-18 binding protein, which is fused to all or a portion of an immunoglobulin. Methods for making immunoglobulin fusion proteins are well known in the art, such as the ones described in WO 01/03737, for example. The person skilled in the art will understand that the resulting fusion protein of the invention retains the biological acitvity of IL-18BP, in particular the binding to IL-18. The fusion may be direct or via a short linker peptide which can be as short as 1 to 3 amino acid residues in length or longer, for example, 13 to 20 amino acid residues in length. Said linker may be a tripeptide of the sequence E-F-M (Glu-Phe-Met), for example, or a 13-amino add linker sequence comprising Glu-Phe-Gly-Ala-Gly-Leu-Val-Leu-Gly-Gly-Gin-Phe-Met introduced between the IL-18BP sequence and the immunoglobulin sequence. The resulting fusion protein has improved properties, such as an extended residence time in body fluids (half-life), increased specific activity, increased expression level, or the purification of the fusion protein is facilitated.

In a preferred embodiment, IL-18BP is fused to the constant region of an Ig molecule. Preferably, it is fused to heavy chain regions, like the CH2 and CH3 domains of human IgG1, for example. The generation of specific fusion proteins comprising IL-18BP and a portion of an immunoglobulin are described in example 11 of WO 99/09063, for example. Other isoforms of Ig molecules are also suitable for the generation of fusion proteins according to the present invention, such as isoforms IgG₂ or IgG₄. or other Ig classes, like IgM or IgA, for example. Fusion proteins may be monomeric or multimeric, hetero- or homomultimeric.

In yet a further embodiment of the invention, an inhibitor of IL-18 is used in combination with a TNF antagonist. TNF antagonists exert their activity in several ways. First, antagonists can bind to or sequester the TNF molecule itself with sufficient affinity and specificity to partially or substantially neutralize the TNF epitope or epitopes responsible for TNF receptor binding (hereinafter termed "sequestering antagonists"). A sequestering antagonist may be, for example, an antibody directed against TNF.

Alternatively, TNF antagonists can inhibit the TNF signaling pathway activated by the cell surface receptor after TNF binding (hereinafter termed "signaling antagonists"). Both groups of antagonists are useful, either alone or together, in combination with an IL-18 inhibitor, in the therapy or prevention of heart diseases.

TNF antagonists are easily identified and evaluated by routine screening of candidates for their effect on the activity of native TNF on susceptive cell lines in vitro, for example human B cells, in which TNF causes proliferation and immunoglobulin secretion. The assay contains TNF formulation at varying dilutions of candidate antagonist, e.g. from 0.1 to 100 times the molar amount of TNF used in the assay, and controls with no TNF or only antagonist (Tucci et al., 1992).

Sequestering antagonists are the preferred TNF antagonists to be used according to the present invention. Amongst sequestering antagonists, those polypeptides that bind TNF with high affinity and possess low immunogenicity are preferred. Soluble TNF receptor molecules and neutralizing antibodies to TNF are particularly preferred. For example, soluble TNF-RI and TNF-RII are useful in the present invention. Truncated forms of these receptors, comprising the extracellular domains of the receptors or functional portions thereof, are more particularly preferred antagonists according to the present invention. Truncated soluble TNF type-I and type-II receptors are described in EP914431, for example.

Truncated forms of the TNF receptors are soluble and have been detected in urine and serum as 30 kDa and 40 kDa TNF inhibitory binding proteins, which are called TBPI and TBPII, respectively (Engelmann et al., 1990). The simultaneous, sequential, or separate use of the IL-18 inhibitor with the TNF antagonist is preferred according to the invention.

In a further preferred embodiment, human soluble TNF-RI (TBPI) is the TNF antagonist to be used according to the invention. The natural and recombinant soluble TNF receptor molecules and methods of their production have been described in the European Patents EP 308 378, EP 398 327 and EP 433 900.

Derivatives, fragments, regions and biologically active portions of the receptor molecules functionally resemble the receptor molecules that can also be used in the present invention. Such biologically active equivalent or derivative of the receptor molecule refers to the portion of the polypeptide, or of the sequence encoding the receptor molecule, that is of sufficient size and able to bind TNF with such an affinity that the interaction with the membrane-bound TNF receptor is inhibited or blocked.

The IL-18 inhibitor can be used simultaneously, sequentially or separately with the TNF inhibitor.

in accordance with the present invention, the medicament may further comprise known agents used for the treatment of heart diseases, such as nitrates, e.g. nitroglycerin, diuretics. ACE Inhibitors, digitalis, beta-Blockers, or Calcium blockers, in combination with an IL-18 inhibitor. The active components may be used simultaneously; sequentially, or separately.

In a further preferred embodiment of the present Invention, the inhibitor of IL-18 is used in an amount of about 0.001 to 100 mg/kg or about 1 to 10 mg/kg or 2 to 5 mg/kg.

The IL-18 inhibitor according to the invention is preferably administered systemically, and preferably subcutaneously or intramuscularly.

The invention further relates to the use of an expression vector comprising the coding sequence of an inhibitor of IL-18 selected from antibodies against IL-18, antibodies against any of the IL-18 receptor subunits, and IL-18 binding proteins, or isoforms, muteins, fused proteins thereof inhibiting the biological activity of IL-18 in the preparation of a medicament for the prevention and/or treatment of cardiomyopathy. Thus, a gene therapy approach is considered in order to deliver the IL-18 inhibitor to the site where it is required. In order to treat and/or prevent a heart disease, the gene therapy vector comprising the sequence of an inhibitor of IL-18 may be injected directly into the diseased tissue, for example, thus avoiding problems Involved in systemic administration of gene therapy vectors, like dilution of the vectors, reaching and targeting of the target cells or tissues, and of side effects.

The use of a vector for inducing and/or enhancing the endogenous production of an inhibitor of IL-18 in a cell normally silent for expression of an IL-18 inhibitor, or which expresses amounts of the inhibitor which are not sufficient wherein the inhibitor of IL-18 is selected from IL-18 binding proteins, or isoforms thereof inhibiting the biological activity of IL-18 are also contemplated according to the invention. The vector may comprise regulatory sequences functional in the cells desired to express the inhibitor or IL-18. Such regulatory sequences may be promoters or enhancers, for example. The regulatory sequence may then be introduced into the right locus of the genome by homologous recombination, thus operably linking the regulatory sequence with the gene, the expression of which is required to be induced or enhanced. The technology is usually referred to as "Endogenous Gene Activation" (EGA), and it is described e.g. in WO 91/09955.

It will be understood by the person skilled in the art that it is also possible to shut down IL-18 expression directly, without using an inhibitor of IL-18, with the same technique. To do that, a negative regulation element, like e.g. a silencing element, may be introduced into the gene locus of IL-18, thus leading to down-regulation or prevention of IL-18 expression. The person skilled in the art will understand that such down-regulation or silencing of IL-18 expression has the same effect as the use of an IL-18 inhibitor In order to prevent and/or treat disease.

The IL-18 inhibitor to be used in accordance with the present invention may be preferable administered as a pharmaceutical composition, optionally in combination with a therapeutically effective amount of a TNF inhibitor.

IL-18BP and its isoforms, muteins, fused proteins, functional derivatives, active fractions or circularly permutated derivatives as described above are the preferred active ingredients of the pharmaceutical compositions.

The definition of "pharmaceutically acceptable" is meant to encompass any carrier, which does not interfere with effectiveness of the biological activity of the active Ingredient and that is not toxic to the host to which it is administered. For example, for parenteral administration, the active protein(s) may be formulated in a unit dosage form for injection in vehicles such as saline, dextrose solution, serum albumin and Ringer's solution.

The active ingredients of the pharmaceutical composition according to the invention can be administered to an individual in a variety of ways. The routes of administration include intradermal, transdermal (e.g. in slow release fonnulations), intramuscular, intraperitoneal, intravenous, subcutaneous, oral, intracranial, epidural, topical, and intranasal routes. Any other therapeutically efficacious route of administration can be used, for example absorption through epithelial or endothelial tissues or by gene therapy wherein a DNA molecule encoding the active agent is administered to the patient (e.g. via a vector), which causes the active agent to be expressed and secreted in vivo. In addition, the protein(s) according to the invention can be administered together with other components of biologically active agents such as pharmaceutically acceptable surfactants, excipients, carriers, diluents and vehicles.

For parenteral (e.g. intravenous, subcutaneous, intramuscular) administration, the active protein(s) can be formulated as a solution, suspension, emulsion or lyophilized powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active protein(s) according to the invention can also be ameliorated by using conjugation procedures which increase the half-life of the molecule in the human body, for example linking the molecule to polyethylenglycol, as described in the PCT Patent Application WO 92/13095.

The therapeutically effective amounts of the active protein(s) will be a function of many variables, including the type of antagonist, the affinity of the antagonist for IL-18, any residual cytotoxic activity exhibited by the antagonists, the route of administration, the clinical condition of the patient (including the desirability of maintaining a non-toxic level of endogenous IL-18 activity).

A "therapeutically effective amount" is such that when administered, the IL-18 inhibitor results in inhibition of the biological activity of IL-18. The dosage administered, as single or multiple doses, to an individual will vary depending upon a variety of factors, including IL-18 inhibitor pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining the inhibition of IL-18 in an individual.

According to the invention, the inhibitor of IL-18 is used in an amount of about 0.001 to 100 mg/kg or about 0.01 to 10 mg/kg or body weight, or about 0.1 to 5 mg/kg of body weight or about 1 to 3 mg/kg of body weight or about 2 mg/kg of body weight.

The route of administration which is preferred according to the invention is administration by subcutaneous route. Intramuscular administration is further preferred according to the invention. In order to administer the IL-18 inhibitor directly to the place of its action, it is also preferred to administer it topically.

In further preferred embodiments, the inhibitor of IL-18 is administered daily or every other day.

The daily doses are usually given in divided doses or in sustained release form effective to obtain the desired results. Second or subsequent administrations can be performed at a dosage which is the same, less than or greater than the initial or previous dose administered to the individual. A second or subsequent administration can be administered during or prior to onset of the disease.

According to the invention, the IL-18 inhibitor can be administered prophylactically or therapeutically to an individual prior to, simultaneously or sequentially with other therapeutic regimens or agents (e.g. multiple drug regimens), in a therapeutically effective amount, in particular with a TNF inhibitor and/or another cardioprotective agent. Active agents that are administered simultaneously with other therapeutic agents can be administered In the same or different compositions.

The invention further relates to a method for the preparation of a pharmaceutical composition comprising admixing an effective amount of an IL-18 inhibitor and/or a TNF antagonist with a pharmaceutically acceptable carrier.

The invention further relates to a method of treatment of a heart disease, comprising administering a pharmaceutically effective amount of an IL-18 inhibitor, optionally in combination with a phamaceutically effective amount of an TNF antagonist, to a patient in need thereof.

### EXAMPLES

### Example 1: Inhibition of IL-18 reduces myocardial ischemic dysfunction in vitro

### Material and Methods

Reagents IL-18BPa isoform was expressed with a N-torminal (His)₆ tag in Chinese hamster ovary cells and purified to homogeneity. The ability of IL-18BPa-(His)₆ to neutralize IL-18 has been described (Kim et al., 2000). The ICE Inhibitor (ICEi) Ac-Try-Val-Ala-Asp-chloromethylketone (YVAO) was purchased from Alexis Biochemicals (San Diego) and solubililized in DMSO at 10 mg/ml. The ICEi was diluted in Tyrode's solution before being used. On human peripheral blood mononuclear cells, the ICEi reduces endotoxin-induced secretion of mature IL-1β by 92%, as measured by ELISA (Cistron Biotechnology, Pine Brook, NJ).

Isolated Atrial Trabeculae Patients undergoing elective coronary artery bypass surgery with a pump oxygenator require insertion of a canula into the right atrium. At that time, a small segment of the right atrial appendage is routinely excised and discarded. Trabeculae were obtained from this discarded tissue. Human atrial tissue was placed in oxygenated modified Tyrode's buffer solution at 4°C. Modified Tyrode's solution was prepared daily with deionized distilled water and contained D-glucose at 5.0 mmol/liter, CaCl₂ at 2.0 mmol/liter, NaCl at 118.0 mmol/liter, KCI at 4.0 mmol/liter, MgSO₄-7H₂O at 1.2 mmol/liter, NaHCO₃ at 25.0 mmol/liter, and NaH₂PO₄ at 1.2 mmol/liter. The substrate-free Tyrode's solution contained choline chloride at 7 mmol/liter to maintain osmolarity. Unless otherwise indicated, chemicals and reagents were obtained from Sigma. Two to four trabeculae (4-7 mm long and <1.0 mm in diameter) were attached to a force transducer and immersed in a heated (37°C) 30-ml bath of modified Tyrode's solution; a 92.5% O₂/7.5% CO₂ mixture was bubbled during normoxia. This gas mixture provided an O₂ partial pressure of >350 mmHg (1 mmHg = 133 Pa), a partial pressure of CO₂ of 36-40 mmHg, and a pH of 7.35-7.45. Each parameterwas checked routinely with an automated blood gas analyzer. The organ bath temperature was maintained at 37°C throughout the experiment. During simulated ischemia, the gas mixture was switched to 92.5% N₂/7.5% CO₂. This mixture produced an O₂ partial pressure of <50 mmHg. The buffer solution was changed every 20 min except during the 30-min period of simulated ischemia.

Experimental Design Trabeculae were equilibrated for 90 min to increase the baseline stretch force to 1,000 mg and to allow stabilization of developed force. Trabeculae that failed to generate more than 250 mg of developed force were excluded from the study. During the 90 min of equilibration, pacing was performed with platinum electrodes (Radnoti Glass, Monrovia, CA) for field stimulation. The electrodes were placed on either side of the trabeculae, stimulated (Grass SD9 stimulator, Warwick, RI) with 6-ms pulses at a voltage 20% above threshold, and paced at 1 Hz during normoxia and at 3 Hz during ischemia. Contractions were monitored by force transducers (Grass FT03) and recorded with a computerized preamplifier and digitizer (MacLab Quad Bridge, MacLab/8e, AD Instruments, Milford, MA) and continuously monitored with a Macintosh computer.

After equilibration, trabeculae from a single patient were studied under three experimental conditions: control conditions consisted of 90 min of normoxic suprafusion; I/R consisted of 30 min of simulated ischemia followed by 45 min of reperfusion; and the third condition consisted of an anticytokine intervention. In the latter case, the anticytokine was added to the suprafusion bath just before the onset of ischemia and was present throughout the 45 min of reperfusion.

Preserved Trabecular CK Activity End reperfusion tissue (90 min) CK activity was determined as described (Kaplan et al., 1993). Tissues were homogenized in 100 vol of ice-cold isotonic extraction buffer (Cleveland et al., 1997, Kaplan et al., 1993). The assay was performed with a CK kit (Sigma) by using an automated spectrophotometer. Results are presented as units of CK activity per mg (wet weight of tissue).

RNA Isolation and Reverse Transcription-Coupled PCR Fresh trabeculae were homogenized in Tri-Reagent (Molecular Research Center, Cincinnati), and total RNA was isolated with chloroform extraction and isopropanol precipitation. The RNA was solubilized in diethyl-pyrocarbonate-treated water, DNase-treated, and quantitated by using GeneQuant (Amersham Pharmacia Biotech). cDNA methods have been described (Reznikov et al., 2000). For each PCR, the following sequence was used: preheat at 95°C for 15 min, then cycles of 94°C for 40 s, 55°C for 45 s, and 72°C for 1 min, with a final extension phase at 72°C for 10 min. The optimal number of cycles was determined as 35. The primers for glyceraldehyde-3-phosphate dehydrogenase (GAPDH) and human IL-18 (Reznikov et al., 2000) and for human IL-18BPa (Kim et al., 2000) have been reported. The PCR products were separated on a 1.5% agarose gel containing 0.5x TBE (50 mM Tris/45 mM boric acid/0.5 mM EDTA, pH 8.3) with ethidium bromide at 0.5 mg/ml, visualized by UV. illumination, and photographed. Densitotmetry was performed on the negative image (IMAGEQUANT software, Molecular Dynamics), and the relative absorbance of the IL-18 and IL-18BP PCR products was corrected against the absorbance obtained for GAPDH.

IL-18 Determinations Fresh trabeculae were homogenized as described above for CK measurements. IL-18 was analyzed with liquid-phase electrochemiluminescence (ECL, lgen, Gaithersburg, MD). Mouse anti-human IL-18 mAb (R &D Systems) was labeled with ruthenium (Igen). In addition, affinity-purified goat anti-human IL-18 antibody (R & D) was labeled with biotin (igen). The biotinylated antibody was diluted to a final concentration of 1 µg/ml in PBS (pH 7.4) containing 0.25% BSA, 0.5% Tween-20, and 0.01% azide (ECL buffer). Per assay tube, 25 µl of the biotinylated antibody was preincubated at room temperature with 25 µl of streptavidin-coated paramagnetic beads (Dynal, Great Neck, NY) at 1 µg/µl for 30 min by vigorous shaking. Samples to be tested (25 µl) or standards were added to tubes followed by 25 µl of ruthenylated antibody (final concentration, 1 µg/µl, diluted in ECL buffer). The tubes were then shaken for 24 h. The reaction was quenched by the addition of PBS at 200 µl per tube and the amount of chemiluminescence was determined with an Origen Analyzer (lgen). The limit of detection for IL-18 is 16 pg/ml.

Confocal Microscopy Human atrial tissue obtained during insertion of the canula of the pump oxygenator was placed in a plastic holder of 1 cm (Meldrum et al., 1998), embedded, and frozen in tissue-freezing medium (Triangle Biomedical Sciences, Durham, NC) on isopentane cooled with dry ice. Frozen sections (5 µm) were cut on a Leica CM 1850 cryostat (Leica, Deerfield, IL). The slides were fixed for 10 min in 4% paraformaldehyde, air-dried, and incubated for 20 min in PBS supplemented with 10% normal goat serum. Sections were incubated in a 1:100 dilution of rabbit anti-human IL-18 antibody (Peprotech, Rocky-Hill, NJ) or nonimmune rabbit IgG at 1 µg/ml as negative control. The antibodies were diluted in PBS containing 1% BSA. After an overnight incubation at 4°C, the sections were washed three times with 0.5% BSA in PBS. The sections were then incubated with a secondary goat anti-rabbit antibody conjugated to Alexa488 (Molecular Probes) for 60 min at room temperature in the dark. Nuclei were stained blue with bisbenzimide (Sigma) at 1 µg/100 ml. After staining, sections were washed and examined with the Leica DM RXA (Leica) confocal laser scanning system and analyzed with SLIDEBOOK software for Macintosh (Intelligent Imaging Innovations, Denver).

Statisical Analysis Data are expressed as the mean ± SEM. Mean changes in developed force were calculated relative to the control value at 90 min for each patient's tissue. Statistical significance of differences between groups were determined by factorial ANOVA with Bonferroni-Dunn post hoc analysis. Statistical analyses were performed with STAT-VIEW 4.51 software (Abacus Concepts, Calabasas, CA).

### Results

### The Effect of Neutralization of Endogenous IL-18 with IL-18BP on Postischemic Developed Force

Fig. 1A demonstrates the kinetic response of trabeculae to I/R injury. The final 15 min of equilibration are shown and normalized to 100% at the beginning of the experimental period. Control trabeculae are suprafused under normoxic conditions throughout the experiment. As shown, there is a reduction (10%) in the developed force in the control trabeculae. Trabeculae subjected to ischemia exhibit a rapid decline in contractile function; on reperfusion, contractile force returns to approximately 25% of the control developed force. In contrast, trabeculae exposed to ischemia but in the presence of IL-18BP returned to 55% of the control developed force. To assess the I/R response of heart tissues from several patients, the level of developed force in the control trabeculae at 90 min was set at 100% for each patient's sample, and the relative percent change in developed force for the experimental groups was calculated.

As shown in Fig. 1*B*, postischemic developed force in untreated trabeculae (I/R) was reduced to a mean of 35% of control. However, in the presence of IL-18BP, this reduction was attenuated to a mean of 66.2% of control at 1 µg/ml and 76% of control at 5 µg/ml, respectively. These results suggest that I/R leads to release of biologically active IL-18 after processing endogenous precursor IL-18 by ICE. Therefore, IL-18 was measured in freshly obtained atrial tissue. As shown in Fig. 2, basal IL-18 was present in trabeculae obtained before the insertion of the of pump-oxygenator canula into the right atrium. After 90 min of equilibration, 30 min of ischemia, and 45 min of reoxygenation, trabeculae were homogenized, and IL-18 levels determined. There was a 4.5-fold increase in IL-18 in the tissue after I/R (Fig.2).

Steady-state mRNA levels for IL-18 and IL-18BP were also determined in these tissues. We observed basal gene expression for IL-18 and IL-18BP in the freshly obtained preischemic atrial homogenates (Fig.3 A, B). Similar to the increase in IL-18 protein, I/R induced a further increase in steady-state IL-18 mRNA levels (4.7-fold increase). IL-18BP gene expression was also observed in freshly obtained atrial tissue and increased only modestly (1.3-fold) after I/R.

Location of IL-18 in Human Myocardium Because IL-18 protein, as measured by ECL, and IL-18 mRNA are present in freshly obtained myocardial homogenates, histochemical staining was used to determine the location of IL-18. Atrial tissues was obtained just before insertion of the pump-oxygenator canula and was immediately snap-frozen (not shown). IL-18 was observed in resident myocardial macrophages and within the vascular endothelial cells. The IL-18 in macrophages and endothelial cells is present before any operation-related ischemia takes place and is present in the absence of contact with any foreign surfaces. The localization of IL-18 in resident macrophages and endothelial cells is consistent with previous studies of constitutive preformed precursor IL-18 in freshly obtained human peripheral monocytes from healthy subjects (Puren et al., 1999). Therefore, it can be concluded that preformed precursor IL-18 exists in the myocardium of patients scheduled for coronary artery bypass for ischemic heart disease.

### The Effect of ICE Inhibition on Postischemic Developed Force

Because IL-18BP effectively attenuated ischemia-induced myocardial dysfunction, we hypothesized that inhibition of the conversion of preformed precursor IL-18 to mature IL-18 would also attenuate ischemia-induced myocardial dysfunction. Therefore, the specific ICE inhibitor YVAD was added to the suprafusion bath before the onset of ischemia. ICE inhibition by the addition of YVAD was continued throughout the ischemic period and during reperfusion. YVAD-mediated inhibition of ICE resulted in attenuation of ischemia-induced myocardial dysfunction, as shown by the improvement in contractile function from 35% of control in I/R to 60% at 10 µg/ml and 75.8% at 20 µg/ml (Fig. 4). These results confirm that biologically active IL-18 in human myocardium is the result of cleavage of preformed precursor IL-18 by ICE. In addition, these results suggest that myocardial ischemia may activate latent ICE.

### Preservation of Cellular Viability

Intracellular levels of CK were used to assess the degree of cellular viability after I/R. In this assay, the higher the CK value, the greater the number of viable cells. Each of the anticytokine interventions resulted in the preservation cellular viability. As demonstrated in Fig. 5, IL-18BP and ICE inhibition (10 and 20 µg/ml), increased intracellular CK levels after I/Rfrom 1,399 to, 5,921, 5,675, 6,624, and 4,662 units of CK activity per mg (wet tissue), respectively. These observations suggest that inhibition of I/R-induced activation of IL-18 and preserves myocellular viability in this ex *vivo* model.

### Effect of neuralization of TNFα induced myocardial function

As shown in Fig. 6, the developed force (DF) of trabeculae was reduced by 18% after 90 minutes of exposure to exogenous TNFα. Neuralization of endogenous iL-18 on contractile function in human myocardium exposed to exogenous TNFα by incubation with IL-188P for ten minutes prior to the addition of TNFα reduced the magnitude of fall in developed force (DF), see Fig. 6. After 90 minutes, the developed force in the contro! group was decreased by 18%, while in the TNFα-exposed trabeculae,it it creased by 58% compared to control. However, in TNFα-exposed trabeculae with IL-18BP, developed force fell by only 30% compared to control. These data suggest that direct effects of TNFα on myocardial contractile depression are mediated, at least in part, by biologically active, endogenous IL-18.

### Effect of exogenous IL-18 on developed force

Next, the direct effect of exogenous IL-18 on myocardial contractile function was determined. IL-18 was added to suprafused trabeculae after 90 minutes of equilibration and with each bath change. As shown in Figure 7, IL-18 leads to a slow but progressive decrease in developed force during the experimental period. After 90 minutes of continuous exposure to IL-18, developed force was decreased by 42%. These data demonstrate that exogenous IL-18, similar to TNFα, acts as a myocardial depressant.

Interestingly, IL-18 is not as potent a myocardial depressant as is TNFα.

### Preservation of cellular viability

Casases are often associated with apoptosis. To assess cellular viability in trabeculae exposed to TNFα, tissue intracellular creatine kinase (CK) was measured. In this assay, high CK levels indicate viable cells. As depicted in Fig. 8, control trabeculae which underwent 90 minutes of normoxic superfusion, contained 6801±276 units of CK activity per milligram of wet tissue weight. In contrast, trabeculae exposed to a 30/45 minute I/R injury or 90 minutes of TNFα exposure exhibited decreased levels of preserved CK of 1774+181 and 3246±217 units/mg, respectively. Trabeculae exposed to TNFα in the presence of IL-18BP contained 5605±212 units/mg of tissue. Interestingly, trabeculae treated with TNFα had greater preserved CK levels compared to I/R trabeculae. This was an unexpected finding since the magnitude of developed force at the end of the experimental period was similar for I/R and TNFα.

### Example 3: IL-18BP protects from myocardial infarction IL-18BP in vivo Method

### In vivo intramuscular electrotransfer of murine IL-18BP expression plasmid

C57BL/6 mice received at 3-week-interval, 3 injections with an expression plasmid containing the cDNA for IL-18BP (called pcDNA3-IL18BP, described in WO 01/85201). The control mice were injected with the control empty plasmid. Murine IL-18BP isoform d cDNA isolated as described (accessory number # Q9ZOM9) (Kim et al., 2000) was subcloned into the EcoR1/Not1 sites of mammalian cell expression vector pcDNA3 under the control of the cytomegalovirus promotor (Invitrogen). Control plasmid was a similar construct devoid of therapeutic cDNA. Control group contained 31 mice, the experimental group receiving IL-18BP comprised 27 mice.

The IL-18BP or control, expression plasmid (60 µg) was injected in both tibial cranial muscles of the anesthetised mouse as previously described (Mallat et al., 1999). Briefly, transcutaneous electric pulses (8 square wave electric pulses of 200 V/cm, 20 msec duration at 2 Hz) were delivered by a PS-15 electropulsator (Genetronics, France) using two stainless steel plate electrodes placed 4.2 to 5.3 mm apart, at each side of the leg.

### Induction of infarction into the left ventricle

Twenty four hours after administration of the IL-18BP plasmid or of empty plasmid, mice were anesthetized by IP injection of xylazine and ketamine, ventilated and subjected to thoracotomy. The left main coronary artery was then permanently ligated using a 8-0 prolene suture, in order to induce myocardial infarction, after which the chest was dosed and the animals were allowed to recovery from anesthesia. Peroperative mortality was less than 20%. Post-operative mortality was 48% in the control group and 26% in the experimental group, and occurred almost exclusively 4-5 days after ligation.

Seven days after ligation, the mice were reanesthetized and left venticular (LV) dimensions were assessed by echocardiography in the dosed chest state, using a ATL HDI 5000 echocardiograph. LV Fractional shortening was calculated from the measured end diastolic and end systolic diameters. At the end of the echocardiographic measurement, the heart was then taken out, fixed, and later cut in sections. Histological sections were then stained with sirius red for the determination of infarct size.

### Results

The diastolic diameter of the left ventricle seven days after ligation in the surviving mice was as follows:

0:53+0.01 mm (n=20) in mice treated with IL-18BP versus 0.59+0.01 mm in control mice (n=16), p < 0.01.

The systolic diameter of the left ventricle seven days after ligation in the surviving mice was as follows:

0.45+0.02) in mice treated with IL-18BP versus 0.52+0.02 in control mice, p < 0.01

Fractional shortening of the left ventricule: 15+1% in mice treated with IL-18BP versus 11+1 % in control mice p < 0.01.

Conclusion: IL-18BP reduces the mortality of mice after myocardial infarction induced by total coronary ligation of the left ventricle by 50 %. In addition to this, the function of the left ventricle was significantly improved, as shown by reduced systolic and diastolic diameters of the left ventricle.

### REFERENCES

1. Bolli, R. (1990) Circulation 82, 723-38.
2. Buggemann et al., Eur. J. lmmunol. 21:1323-1326 (1991)
3. Cain, B. S., Meldrum, D. R., Dinarello, C. A., Meng, X., Banerjee, A. & Harken, A. H. (1998) J Surg Res 76, 117-23.
4. Cleveland, J. C. J., Meldrum, D. R., Cain, B. S., Banerjee, A. & Harken, A. H. (1997) Circulation 96, 29-32.
5. Daemen MA, van't Veer C, Wolfs TG, et al: Ischemia/reperfusion-induced IFN-gamma up-regulation: involvement of IL- 12 and IL-18. J.Immunol. 162:5506-5510,1999
6. DiDonato, J A, Hayakawa, M, Rothwarf, D M, Zandi, E and Karin, M. (1997). Nature 388, 16514-16517.
7. Elliott, M.J., Maini, R.N., Feldmann, M., Long-Fox, A., Charles, P., Bijl, H., and Woody, J.N., 1994, Lancet 344, 1125-1127.
8. Engelmann, H., Novick, D., and Wallach, D., 1990, J.Biol.Chem. 265, 1531-1536.
9. Fantuzzi, G., A. J. Purenl M. W. Harding, D. J, Livingston, and C. A. Dinarello. Blood 91:2118-25, 1998.
10. Grantham (1974), Science, 185. 862-864.
11. Gurevitch, J., Frolkis, I., Yuhas, Y., Paz, Y., Matsa, M., Mohr, R. & Yakirevich, V. (1996) J Am Coll Cardiol 28, 247-52.
12. Herskowitz, A., Choi, S., Ansari, A. A. & Wesselingh, S. (1995) Am J Pathol 146, 419-28.
13. Hochholzer, 9., G. B. Lipford, H. Wagner, K. Pfeffer, and K. Heeg. Infect lmmun. 68: 3502
14. Kaplan, L. J., Blum, H., Banerjee, A. & Whitman, G. J. (1993) J Surg Res 54, 31 1-5.
15. Kim SH, Eisenstein M, Reznikov L, Fantuzzi G, Novick D, Rubinstein M, Dinarello CA. Structural requirements of six naturally occurring isoforms of the IL-18 binding protein to inhibit IL-18. Proc Natl Acad Sci U S A 2000;97:1190-1195.
16. Kim SH et al., J. lmmuno. 2001, 166, pp. 148 -154.
17. Knight DM, Trinh H, Le J, Siegel S, Shealy D, McDonough M, Scallon B, Moore MA, Vilcek J, Daddona P, et al. Construction and initial characterization of a mouse-human chimeric anti-TNF antibody. Mol Immunol 1993 Nov 30:16 1443-53
18. Maniatis et al., Molecular Cloning: A Laboratory Manual, Cold Spring Harbor Laboratory, New York, 1982.
19. Meldrum, D. R., Cleveland, J. C., Jr., Cain, B. S., Meng, X. & Harken, A. H. (1998) Ann Thorac Surg 65, 439-43.
20. Mendez, M.M., Green, L.L., Corvalan, J.R.F., Jia X-C., Maynard-Currie, E.E., Yang, X-D., Gallo, M.L., Louie, D.M., Lee, D.V., Erickson; K.L., Luna, J., Roy, C.M-N., Abderrahim, H., Kirshenbaum, F., Noguchi, M., Smith, D.M., Fukushima, A., Hales, J.F., Finer, M.H., Davis, C.G., Zsebo, K.M. and Jakobovits, A. (1997). "Functional transplant of megabase human immunoglobulin loci recapitulates human antibody response in mice". Nature Genetics, 15 146-56.
21. Nakamura, K, Okamura, H, Nagata, K and Tamura, T. (1989). Infect. Immun. 57, 590-595.
22. Novick, D, Kim, S-H, Fantuzzi, G, Reznikov, L, Dinarello, C, and Rubinstein, M (1999). Immunity 10, 127-136.
23. Parnet, P, Garka, K E, Bonnert, T P, Dower, S K, and Sims, J E. (1996), J. Biol. Chem. 271, 3967-3970.
24. Puren, A. J. , Fantuzzi, G. & Dinarello, C. A. (1999) Proc. Natl. Acad. Sci. USA 96, 2256-2261
25. Raebum, C. D., C. M' Calkins, M. A. Zimmerman, Y. Song, L Ao, A. Banerjee, X. keng, and A. H. Harken. Surgery 130: 319-25., 2001.
26. Reznikov, L. L., Kim, S. H., Westcott, J. Y., Frishman, J., Fantuzzi, G., Novick, D., Rubinstein, M. & Dinarello, C. A. (2000) Proc Natl Acad Sci U S A 97, 2174-2179.
27.Torigoe, K., Ushio, S., Okura, T.; Kobayashi, S., Taniai, M., Kunikate, T., Murakami, T., Sanou, O., Kojima, H., Fuji, M., Ohta, T., Ikeda, M., Ikegami, H. & Kurimoto, M. (1997) J Biol Chem272, 25737-25742.
28. Tomizuka et al., Proc. Natl. Acad. Sci. USA 97:722-727 (2000)
29. Tucci, A., James, H., Chicheportiche, R., Bonnefoy, J.Y., Dayer, J.M., and Zubler, R.H., 1992, J.Immunol. 148, 2778-2784.
30. Yoshimoto T, Takeda, K, Tanaka, T, Ohkusu, K, Kashiwamura, S, Okamura, H, Akira, S and Nakanishi, K (1998). J. Immunol. 161, 3400-3407.

## Claims

1. Use of an inhibitor of IL-18 in the manufacture of a medicament for the treatment and/or prevention of cardiomyopathy, wherein the inhibitor of IL-18 is selected from an inhibitor of caspase-1 (ICE), antibodies against IL-18, antibodies against any of the IL-18 receptor subunits, and IL-18 binding proteins, or isoforms, muteins, fused proteins, or functional derivatives thereof inhibiting the biological activity of IL-18.

2. The use according to claim 1, wherein the inhibitor of IL-18 is an antibody directed against IL-18.

3. The use according to claim 1, wherein the inhibitor of IL-18 is an antibody directed against IL-18 receptor α.

4. The use according to claim 1, wherein the inhibitor of IL-18 is an antibody directed against IL-18 receptor β.

5. The use according to any of the preceding claims, wherein the antibody is a humanized or human antibody.

6. The use according to claim 1, wherein the ICE inhibitor is Ac-Tyr-Val-Ala-Asp-chloromethylketone (YVAD).

7. The use according to claim 1, wherein the inhibitor of IL-18 is an IL-18 binding protein, or an isoform, a mutein, fused protein, or functional derivative thereof inhibiting the biological activity of IL-18.

8. Use according to claim 6, wherein the IL-18 inhibitor is glycosylated at one or more sites.

9. Use according to claim 6 or 7, wherein the fused protein comprises an immunoglobulin (Ig) fusion.

10. Use according to any of claims 6 to 8, wherein the functional derivative comprises at least one moiety attached to one or more functional groups, which occur as one or more side chains on the amino acid residues.

11. Use according to claim 9, wherein the moiety is a polyethylene moiety.

12. The use according to any of the preceding claims, wherein the medicament further comprises a Tumor Necrosis Factor (TNF) antagonist.

13. The use according to claim 11, wherein the inhibitor of IL-18 is used simultaneously, sequentially, or separately with the TNF antagonist.

14. The use according to claim 11 or 12, wherein the TNF antagonist is TBPI and/or TBPII.

15. The use according to any of the preceding claims, wherein the inhibitor of IL-18 is used in a concentration ranging between about 0.001 to 100 mg/kg or about 1 to 10 mg/kg or 2 to 5 mg/kg.

16. Use of an expression vector comprising the coding sequence of an inhibitor of IL-18 in the preparation of a medicament for the treatment and/or prevention of cardiomyopathy, wherein the inhibitor of IL-18 is selected from antibodies against IL-18, antibodies against any of the IL-18 receptor subunits, and IL-18 binding proteins, or isoforms, muteins, fused proteins thereof inhibiting the biological activity of IL-18.

17. Use of an expression vector for inducing and/or enhancing the endogenous production of an inhibitor of IL-18 in a cell in the preparation of a medicament for the treatment and/or prevention of cardiomyopathy, wherein the inhibitor of IL-18 is selected from IL-18 binding proteins, or isoforms thereof inhibiting the biological activity of IL-18.

## Patentansprüche

1. Verwendung eines Inhibitors von IL-18 bei der Herstellung eines Medikaments zur Behandlung und/oder Verhinderung von Kardiomyopathie, worin der Inhibitor von IL-18 ausgewählt ist aus einem Inhibitor von Caspase-1 (ICE), Antikörpern gegen IL-18, Antikörpern gegen eine beliebige der IL-18-Rezeptoruntereinheiten und IL-18-Bindungsproteine, oder Isoformen, Muteinen, fusionierten Proteinen oder funktionellen Derivaten davon, die die biologische Aktivität von IL-18 inhibieren.

2. Verwendung nach Anspruch 1, worin der Inhibitor von IL-18 ein Antikörper ist, der gegen IL-18 gerichtet ist.

3. Verwendung nach Anspruch 1, worin der Inhibitor von IL-18 ein Antikörper ist, der gegen den IL-18-Rezeptor α gerichtet ist.

4. Verwendung nach Anspruch 1, worin der Inhibitor von IL-18 ein Antikörper ist, der gegen den IL-18-Rezeptor β gerichtet ist.

5. Verwendung nach einem der vorhergehenden Ansprüche, worin der Antikörper ein humanisierter oder humaner Antikörper ist.

6. Verwendung nach Anspruch 1, worin der ICE-Inhibitor Ac-Tyr-Val-Ala-Asp-Chlormethylketon (YVAD) ist.

7. Verwendung nach Anspruch 1, worin der Inhibitor von IL-18 ein IL-18-Bindungsprotein oder eine Isoform, ein Mutein, fusioniertes Protein oder ein funktionelles Derivat davon ist, welches die biologische Aktivität von IL-18 inhibiert.

8. Verwendung nach Anspruch 6, worin der IL-18-Inhibitor an einer oder mehreren Stellen glykosyliert ist.

9. Verwendung nach Anspruch 6 oder 7, worin das fusionierte Protein eine Immunglobulin(IG)fusion umfasst.

10. Verwendung nach einem der Ansprüche 6 bis 8, worin das funktionelle Derivat mindestens einen Rest umfasst, der gebunden ist an eine oder mehrere funktionelle Gruppen, die als eine oder mehrere Seitenketten auf den Aminosäureresten auftreten.

11. Verwendung nach Anspruch 9, worin der Rest ein Polyethylenrest ist.

12. Verwendung nach einem der vorhergehenden Ansprüche, worin das Medikament weiterhin einen Tumor-Nekrosefaktor (TNF)-Antagonisten umfasst.

13. Verwendung nach Anspruch 11, worin der Inhibitor von IL-18 gleichzeitig, sequentiell oder getrennt mit dem TNF-Antagonisten verwendet wird.

14. Verwendung nach Anspruch 11 oder 12, worin der TNF-Antagonist TBPI und/oder TBPII ist.

15. Verwendung nach einem der vorhergehenden Ansprüche, worin der Inhibitor von IL-18 in einer Konzentration im Bereich zwischen etwa 0,001 bis 100 mg/kg oder etwa 1 bis 10 mg/kg oder 2 bis 5 mg/kg verwendet wird.

16. Verwendung eines Expressionsvektors, umfassend die codierende Sequenz eines Inhibitors von IL-18 bei der Herstellung eines Medikaments zur Behandlung und/oder Verhinderung einer Kardiomyopathie, worin der Inhibitor von IL-18 ausgewählt ist aus Antikörpern gegen IL-18, Antikörpern gegen eine beliebige der II-18-Rezeptoruntereinheiten und IL-18-Bindungsproteine oder lsoformen, Muteine, fusionierte Proteine davon, die die biologische Aktivität von IL-18 inhibieren.

17. Verwendung eines Expressionsvektors zum Induzieren und/oder Verstärken der endogenen Produktion eines Inhibitors von IL-18 in einer Zelle bei der Herstellung eines Medikaments zur Behandlung und/oder Verhinderung einer Kardiomyopathie, worin der Inhibitor von IL-18 ausgewählt ist aus IL-18-Bindungsproteinen oder Isoformen davon, die die biologische Aktivität von IL-18 inhibieren.

## Revendications

1. Utilisation d'un inhibiteur d'IL-18 dans la fabrication d'un médicament pour le traitement et/ou la prévention d'une cardiomyopathie, dans laquelle l'inhibiteur d'IL-18 est choisi parmi un inhibiteur de caspase-1 (ICE), des anticorps contre 1'IL-18, des anticorps contre n'importe quelles sous-unités du récepteur d'IL-18, et des protéines fixatrices de l'IL-18, ou des isoformes, mutéines, protéines condensées ou des dérivés fonctionnels de celles-ci, inhibant l'activité biologique de l'IL-18.

2. Utilisation selon la revendication 1, dans laquelle l'inhibiteur d'IL-18 est un anticorps dirigé contre l'IL-18.

3. Utilisation selon la revendication 1, dans laquelle l'inhibiteur d'IL-18 est un anticorps dirigé contre le récepteur α d'IL-18.

4. Utilisation selon la revendication 1, dans laquelle l'inhibiteur d'IL-18 est un anticorps dirigé contre le récepteur β d'IL-18.

5. Utilisation selon l'une quelconque des revendications précédentes, dans laquelle l'anticorps est un anticorps humain ou humanisé.

6. Utilisation selon la revendication 1, dans laquelle l'inhibiteur ICE est la Ac-Tyr-Val-Ala-Asp-chlorométhylcétone (YVAD).

7. Utilisation selon la revendication 1, dans laquelle l'inhibiteur de l'IL-18 est une protéine fixatrice d'IL-18, ou un isoforme, une mutéine, une protéine condensée, ou un dérivé fonctionnel de celle-ci inhibant l'activité biologique de 1' IL-18.

8. Utilisation selon la revendication 6, dans laquelle l'inhibiteur d'IL-18 est glycosylé en un ou plusieurs sites.

9. Utilisation selon la revendication 6 ou 7, dans laquelle la protéine condensée comprend une fusion d'immunoglobuline (Ig).

10. Utilisation selon l'une quelconque des revendications 6 à 8, dans laquelle le dérivé fonctionnel comprend au moins un fragment attaché à un ou plusieurs groupes fonctionnels , qui apparaît sous la forme d'une ou plusieurs chaînes latérales sur les résidus d'amino-acides.

11. Utilisation selon la revendication 9, dans laquelle le fragment est un fragment polyéthylène.

12. Utilisation selon l'une quelconque des revendications précédentes, dans laquelle le médicament comprend en outre un antagoniste du facteur de nécrose tumorale (TNF).

13. Utilisation selon la revendication 11, dans laquelle l'inhibiteur d'IL-18 est utilisé simultanément, successivement ou séparément avec l'antagoniste du TNF.

14. Utilisation selon la revendication 11 ou 12, dans laquelle l'antagoniste du TNF est TBPI et/ou TBPII.

15. Utilisation selon l'une quelconques des revendication précédentes, dans laquelle l'inhibiteur d'IL-18 est utilisé à une concentration se situant entre environ 0,001 à 100 mg/kg ou d'environ 1 à 10 mg/kg ou de 2 à 5 mg/kg.

16. Utilisation d'un vecteur d'expression comprenant la séquence codante d'un inhibiteur d'IL-18 dans la préparation d'un médicament pour le traitement et/ou la prévention d'une cardiomyopathie, dans laquelle l'inhibiteur d'IL-18 est choisi parmi des anticorps contre l'IL-18, des anticorps contre n'importe quelles sous-unités du récepteur d'IL-18, et des protéines fixatrices de l'IL-18; ou des isoformes, mutéines, protéines condensées de celles-ci, inhibant l'activité biologique de l'IL-18.

17. Utilisation d'un vecteur d'expression pour induire et/ou accroître la production endogène d'un inhibiteur d'IL-18 dans une cellule dans la préparation d'un médicament pour le traitement et/ou la prévention d'une cardiomyopathie, dans laquelle l'inhibiteur de l'IL-18 est choisi parmi des protéines fixatrices d'IL-18, ou ses isoformes inhibant l'activité biologique de l'IL-18.
